# EUROPEAN PATENT SPECIFICATION

(11) **EP 4 199 865 B1**
(45) Date of publication and mention of the grant of the patent: **30.09.2026**
(21) Application number: 21859266.5
(22) Date of filing: 23.08.2021
(51) Int. Cl.: A61F 2/42, A61B 17/15, A61B 17/92, A61F 2/46

(54) **IMPLANT FOR FOCAL TALUS DEFECTS**
IMPLANTAT FÜR FOKALE TALUSDEFEKTE
IMPLANT POUR DÉFAUTS ASTRAGALIENS FOCAUX

(30) Priority: 21.08.2020 US 202063068425 P
(43) Date of publication of application: 28.06.2023
(73) Proprietor: Paragon Advanced Technologies, Inc., Englewood, CO 80112 (US)
(72) Inventor: PERLER, Adam D., St. Petersburg, Florida 33704 (US); KOWALCZYK, Gregory J., Englewood Colorado, 80112 (US); KENNISON, Carissa E., Cary, North Carolina 27519 (US); DEORIO, James K., Durham, North Carolina 27703 (US); BERTOLOTTI, Luciano, Englewood Colorado, 80112 (US)
(74) Representative: Murgitroyd & Company
(86) International application number: PCT/US2021/047117
(87) International publication number: WO 2022/040623

(56) References cited:
- CN-A- 108 210 130
- CN-U- 208 989 260
- CN-U- 209 316 156
- US-A1- 2015 045 902
- US-A1- 2016 262 903
- US-A1- 2019 350 717
- US-A1- 2019 358 046
- US-A1- 2020 129 311
- US-B2- 10 016 811

## Description

### TECHNICAL FIELD

The present invention relates generally to general, podiatric, and orthopaedic surgery related to the repair of focal defects in a bone. More specifically, but not exclusively, the present invention relates to focal defects in a talus of an ankle joint.

### BACKGROUND OF THE INVENTION

At least one embodiment of the invention relates to an implant used to repair a focal talus defect and a method for inserting this implant into a focal talus defect. The talus bone is positioned in the lower part of an ankle joint. It can bear the weight of an individual as the weight of the individual is transmitted from the person's leg to their foot. Portions or all of a talus bone can become compromised by disease or injury. Therefore, there is a need for an implant for a talus bone, in particular there is a need for a focal talus or talar implant and a method for efficiently implanting this implant into a patient's talus. US2019/358046A1, CN208989260U and CN209316156U disclose related art.

### SUMMARY

The invention is defined in claim 1.

The invention may be used in a method for implanting a talus implant that may include identifying a damaged boney area, projecting a missing damaged area on a contralateral joint and then printing an implant based upon a mirror image of a normal portion of the contralateral joint. The method may also include using at least one guide, removing at least a portion of a damaged region of the talus and then inserting the talar implant and setting the talar implant.

### BRIEF DESCRIPTION OF THE DRAWINGS

The accompanying drawings, which are incorporated in and constitute a part of the specification, illustrate embodiments of the invention and together with the detailed description herein, serve to explain the principles of the invention. It is emphasized that, in accordance with the standard practice in the industry, various features are not drawn to scale. In fact, the dimensions of the various features may be arbitrarily increased or reduced for clarity of discussion. The drawings are only for purposes of illustrating preferred embodiments and are not to be construed as limiting the invention.
**FIG. 1A** is a top perspective view of an implant implanted into a talus, in accordance with an aspect of the present invention;
**FIG. 1B** is a top view of the implant of FIG. 1 implanted into the talus, in accordance with an aspect of the present invention;
**FIG. 2A** is a side perspective view of the implant of FIG. 1 positioned above the talus prior to implantation, in accordance with an aspect of the present invention;
**FIG. 2B** is a side view of the implant of FIG. 1, in accordance with an aspect of the present invention;
**FIG. 2C** is a top view of the implant of FIG. 1, in accordance with an aspect of the present invention;
**FIG. 2D** is a top perspective view of the implant of FIG. 1, in accordance with an aspect of the present invention;
**FIG. 3** is a medial view of the ankle joint with the talus implant of FIG. 1 implanted into the talus bone, in accordance with an aspect of the present invention;
**FIG. 4A** is a side perspective view of an impactor instrument for implanting an focal talar implant;
**FIG. 4B** is another side perspective view of the impactor instrument of FIG. 4A;
**FIG. 4C** is a side perspective view of the impactor instrument of FIG. 4A;
**FIG. 4D** is a side view of the impactor instrument of FIG. 4A;
**FIG. 4E** is another side view of the impactor instrument of FIG. 4A;
**FIG. 4F** is a perspective view of the impactor instrument of FIG. 4A;
**FIG. 5A** is a side view of a reamer;
**FIG. 5B** is a side perspective view of the reamer of FIG. 5A;
**FIG. 5C** is a side view of the reamer of FIG. 5A;
**FIG. 5D** is a front perspective view of the reamer of FIG. 5A;
**FIG. 5E** is an end side view of the reamer of FIG. 5A;
**FIG. 6A** is a perspective view of a custom tibial guide;
**FIG. 6B** is a side perspective view of the custom tibial guide of FIG. 6A;
**FIG. 6C** is a perspective top view of the custom tibial guide of FIG. 6A;
**FIG. 6D** is a top view of the custom tibial guide of FIG. 6A;
**FIG. 7A** is a side view of a custom talar guide;
**FIG. 7B** is a bottom side of the custom talar guide of FIG. 7A;
**FIG. 7C** is a top perspective view of the custom talar guide of FIG. 7A;
**FIG. 7D** is a side view of the custom talar guide of FIG. 7A;
**FIG. 8A** is a perspective view of a custom reamer;
**FIG. 8B** is a top view of the custom reamer of FIG. 8A;
**FIG. 9A** is a perspective view an ankle joint showing a step for inserting an implant in a talus;
**FIG. 9B** is a perspective view an ankle joint showing another step for inserting an implant in a talus;
**FIG. 9C** is a side view of an ankle joint showing another step for inserting an implant in a talus;
**FIG. 9D** is an anterior view of an ankle joint showing another step for inserting an implant in a talus;
**FIG. 9E** is side view of an ankle joint showing another step for inserting an implant in a talus;
**FIG. 9F** is a side view oof an ankle joint showing another step for inserting an implant in a talus;
**FIG. 10** is a flow chart for the method of fabricating and implanting the implant in the talus.

### DETAILED DESCRIPTION FOR CARRYING OUT THE INVENTION

Generally stated, disclosed herein are guides, implants, devices, instruments, systems, and assemblies for achieving bone fusion. Further, methods for using the guides, implants, devices, instruments, systems, and assemblies to repair bone defects are discussed.

In this detailed description and the following claims, the words proximal, distal, anterior or plantar, posterior or dorsal, medial, lateral, superior and inferior are defined by their standard usage for indicating a particular part or portion of a bone or implant according to the relative disposition of the natural bone or directional terms of reference. For example, "proximal" means the portion of a device or implant nearest the torso, while "distal" indicates the portion of the device or implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure. Further, specifically in regards to the foot, the term "dorsal" refers to the top of the foot and the term "plantar" refers the bottom of the foot.

Similarly, positions or directions may be used herein with reference to anatomical structures or surfaces. For example, as the current implants, devices, instrumentation and methods are described herein with reference to use with the bones of the foot, the bones of the foot, ankle and lower leg may be used to describe the surfaces, positions, directions or orientations of the implants, devices, instrumentation and methods. Further, the implants, devices, instrumentation and methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the implants, devices, instrumentation and methods, and the aspects, components, features and the like thereof, described and/or illustrated herein may be changed, varied, modified, reconfigured or otherwise altered for use or association with another side of the body for a same or similar purpose without departing from the scope of the invention, which is defined by the claims. For example, the implants, devices, instrumentation and methods, and the aspects, components, features and the like thereof, described herein with respect to the right foot may be mirrored so that they likewise function with the left foot. Further, the implants, devices, instrumentation and methods, and the aspects, components, features and the like thereof, disclosed herein are described with respect to the foot for brevity purposes, but it should be understood that the implants, devices, instrumentation and methods may be used with other bones of the body having similar structures.

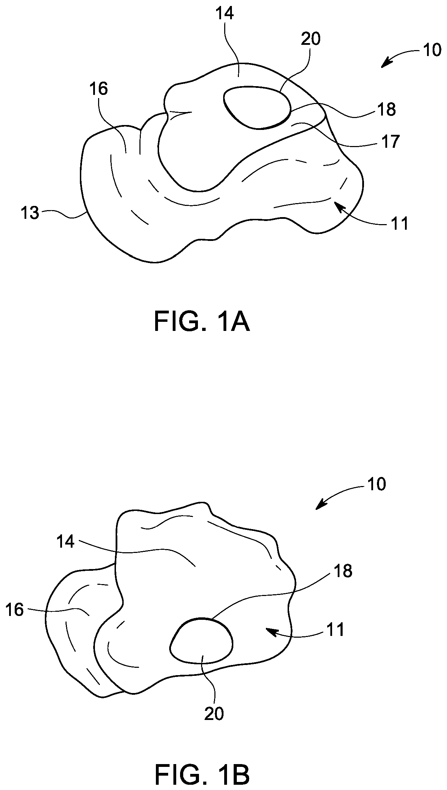

Referring to the drawings, wherein like reference numerals are used to indicate like or analogous components throughout the several views, and with particular reference to FIG. 1A is a top side perspective view of the inventive implant 20 implanted into a talus.

FIG. 1B is a top view of the implant 20 implanted into the talus bone 11. For example, there is shown an implant body 20 implanted around an open rim of a talus bone 11. The talus bone includes a body 17, a neck 16 which leads to a head 13 and a process 14. This feature is shown in FIG. 1A as well.

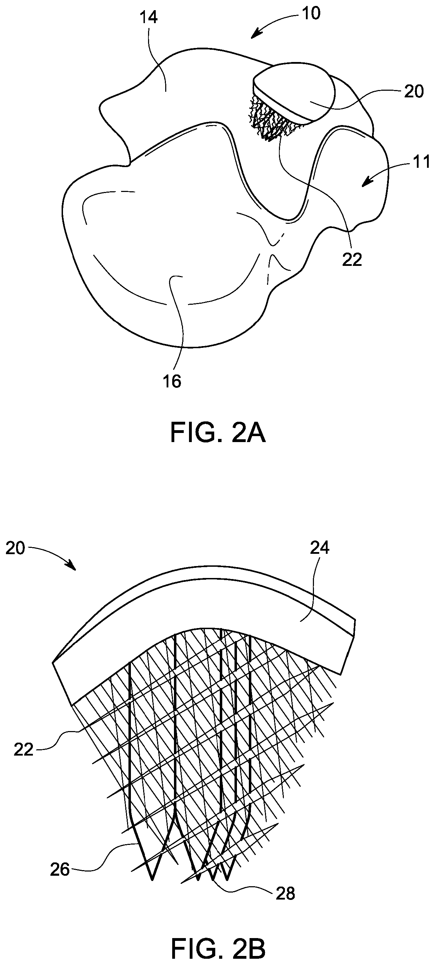

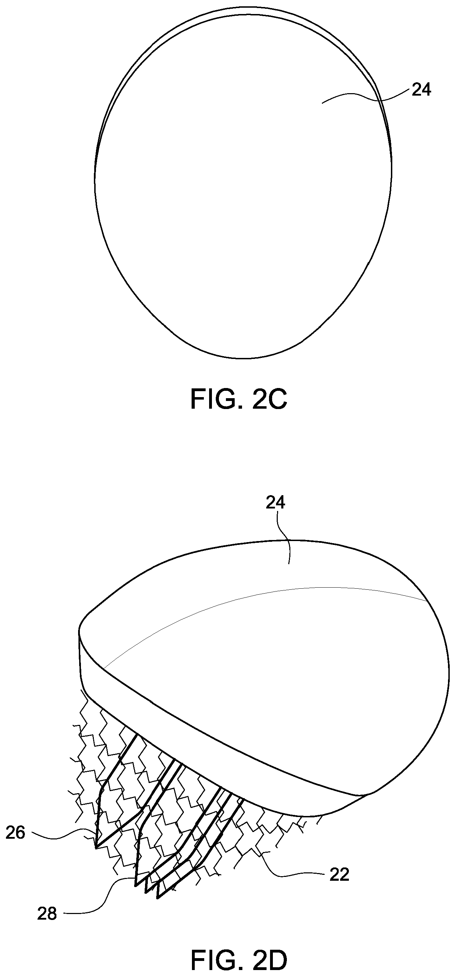

FIG. 2A is a side perspective view of the implant 20 positioned above the talus, wherein the talus implant 20 includes a mesh region 22 having a patterned mesh that can be for example in the form of a honeycomb pattern of varying porosity or alternatively, of a standard or uniform porosity. A plurality of solid prongs such as prongs 26 and 28 (See FIG. 2B) extend out from implant 20 through the mesh and towards a spike end. The implant 20 is configured to be printed such as through 3D printing with the top surface 24 of implant 20 being further coated with a shiny or resilient coat 24 (See FIGS. 2C and 2D).

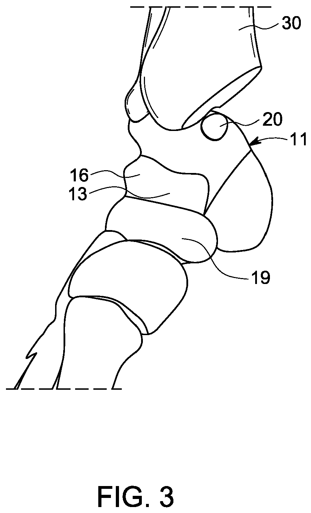

FIG. 3 is a side view of the ankle joint with the talus implant 20 implanted into the talus bone. For example, there is shown the talus 11 having the implant 20 positioned therein. A tibia bone 30 is positioned superior to the talus implant 20 and the talus bone 11. In addition, as shown, the neck of the talus 16 extends into a head 13 which then extends into contact with the navicular bone 19.

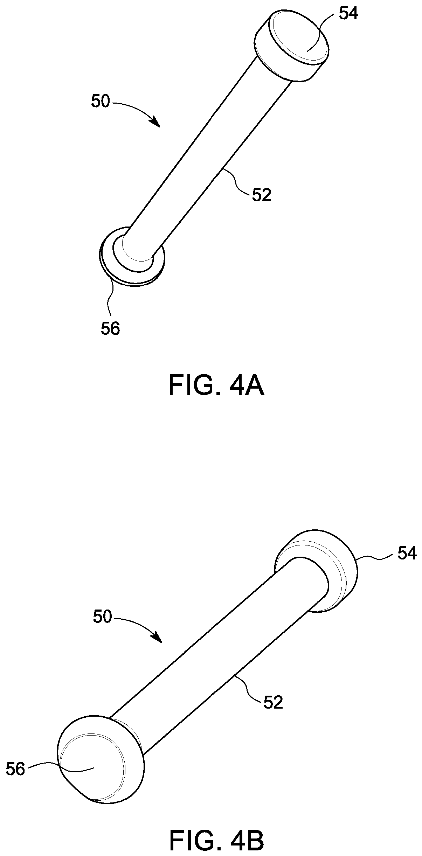

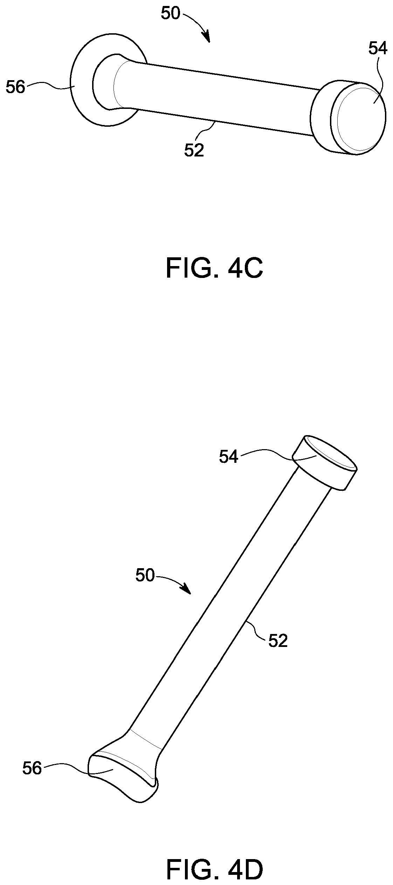

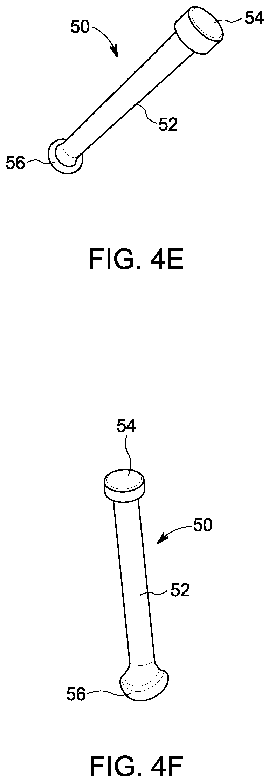

FIGS. 4A-4F show various views of an impactor instrument for implanting the implant 20. The impactor 50 is configured as a tool for inserting the implant 20 into the talus 11. For example, the impactor 50 includes a body section 52, a first end 54 and a second end 56 (See FIGS. 4B, 40 and 4F). The second end 56 is a cup shape (or concave bowl shape) configured to accept the head or surface of the implant 20. FIGS. 4C, 4D, 4E and 4F are 2-D wireframe depictions of the impactor 50 from various perspectives.

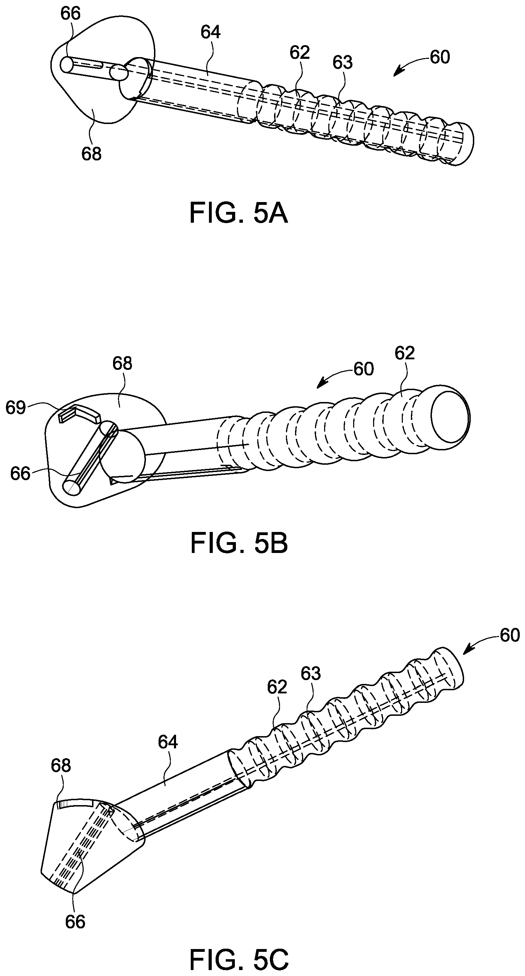

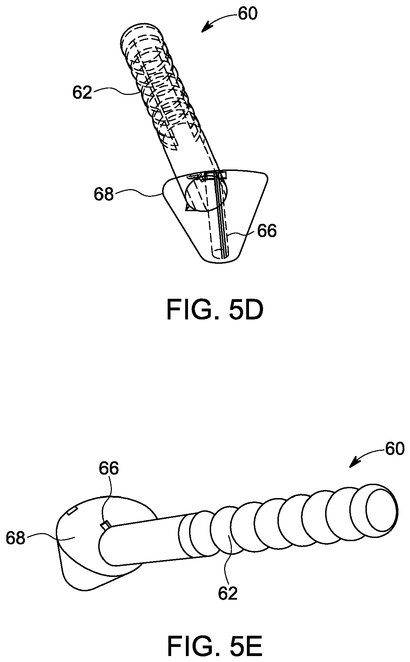

FIGS. 5A-5E show different views of a bone cutting reamer. For example, the reamer is configured to carve out a section of the talus 11 to facilitate the implantation of the implant 20. The reamer 60 includes a handle 62, having indents 63. The handle has a smooth or continuous section 64 that is proximate and extends to a cutting head 68 This handle extends to a head 68. The head 68 is configured to be a frusto-conical shape so that it can be extended into the talus 11 to create an opening that facilitate the implantation of the implant 20. The head also includes an open channel 66 which extends substantially longitudinally along the handle 62. This open channel 66 allows for the withdrawal of material or fluid from the talus 11 when it is being actuated.

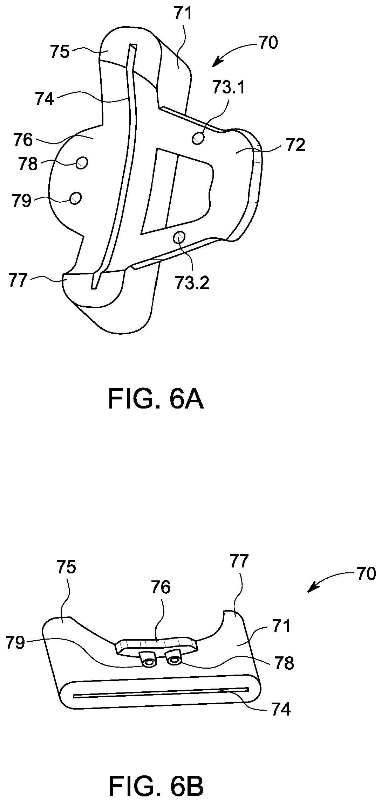

FIG. 6A is a perspective view of a custom tibial cutting guide 70 for which the tibial guide 70 includes a body section 71 with wings 75 and 77 with an extension section 72 having holes 73.1 and 73.2. There is also aprotrusion 76 having holes 78 and 79. In addition, a slot 74 extends along the width of the tibial cutting guide through which a saw blade may be placed.

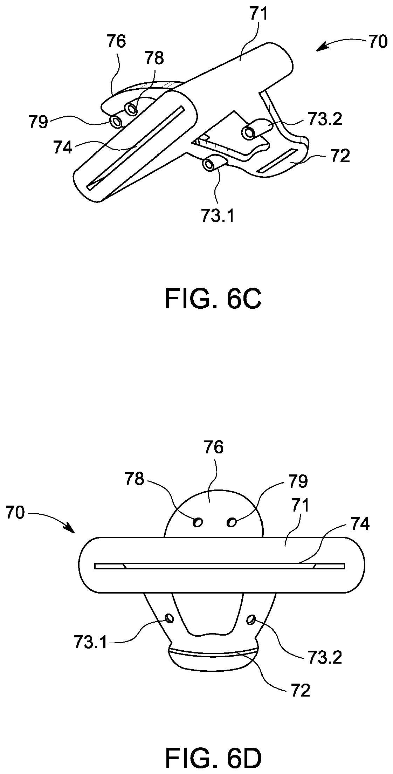

FIG. 6B is a side perspective view of the tibial cutting guide 70 and more clearly shows the body section 71 having the protrusion 76 with the holes 78 and 79 extending down in a cylindrical manner. In addition, FIG. 6B shows the wings 75 and 77. The cutting slot 74 is shown to extend from a first side shown in FIG. 6A to the second side shown in FIG. 6B (see also FIGS. 6C and 6D as well). The tibial cutting guide 70 is configured to be attached in close proximity to the tibia to allow a medical practitioner to slice or remove a portionof a patient's tibia through a knife or other cutting mechanism operating through the slot 74.

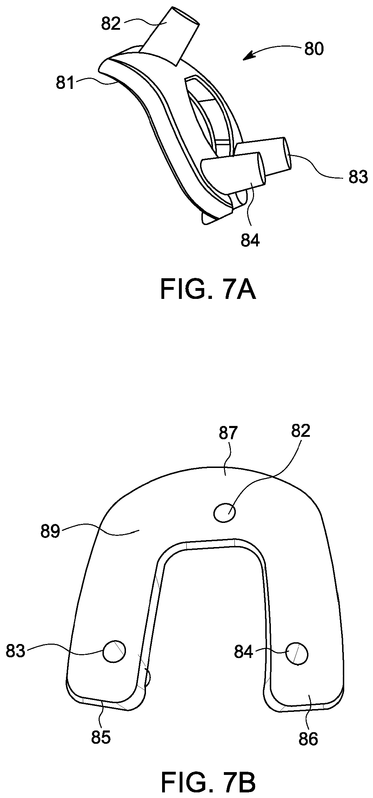

FIG. 7A is a side view of a custom talar cutting guide 80, which includes holes 82, 83 and 84 disposed in a U-shaped body 81. These holes 82, 83, and 84 extend in a cylindrical manner to form slots for receiving pins which are insertable into these holes and which are insertable into a corresponding talus implant.

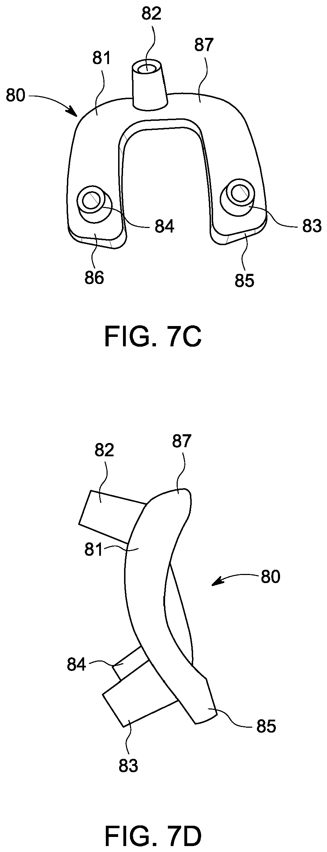

FIG. 7B shows the bottom side for the talar cutting guide 80. The surface of the bottom side is configured to be placed on top of the talus 11 prior to a reaming step. Accordingly, the opposite facing top side of the talar cutting guide 80 which has holes 82, 83 and 84 which are configured to receive pins is shown in FIG. 7C. The body 81 of the talar cutting guide is shaped as a substantially U- shape or horseshoe shape having a first end 86 and a hole 84, a second end 85 having a hole 83 and a distal region 87 spaced opposite the ends 85 and 86. FIG. 7D is a side view of the custom talar cutting guide 80 which includes holes 82, 83 and 84. Hole 82 is positioned in the distal region 87 of the body 81.

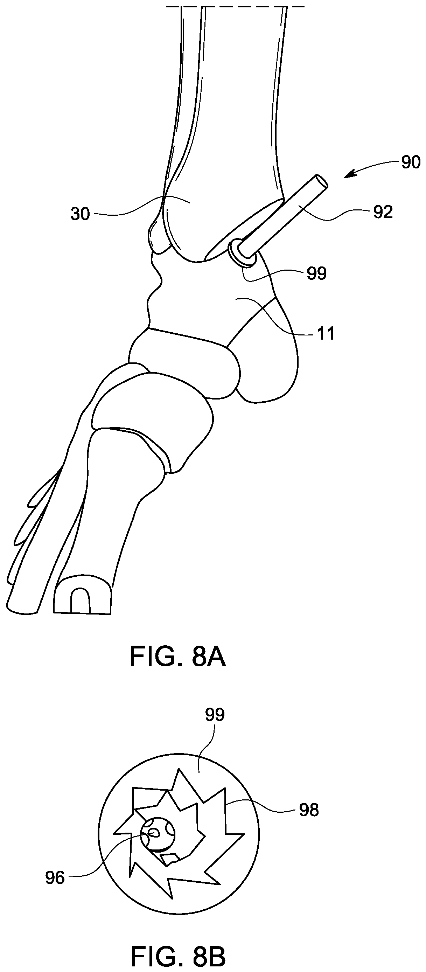

FIG. 8A is a perspective view of a custom bone cutting reamer 90 which is positioned in talus 11 and configured to remove bone from a region of the talus 11 by using a head 99, and a shaft 92 which is integrally attached to the head 99. The head 99 has a plurality of teeth 98 as well as a central hole 96. The central hole 96 (See FIG. 8B) can be used to slide over a guide pin which can be set by the talus guide. (See FIG. 7B).

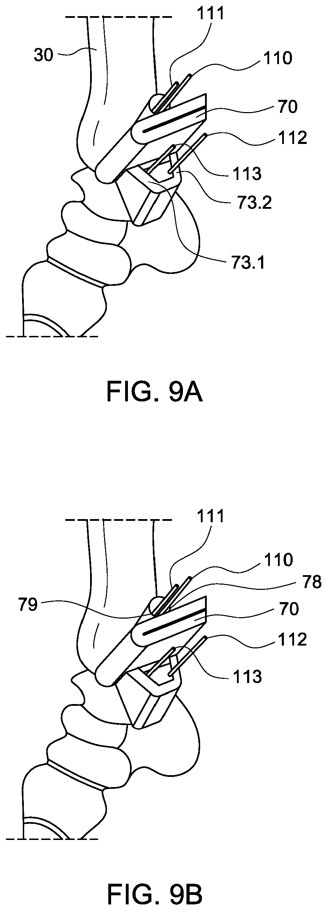

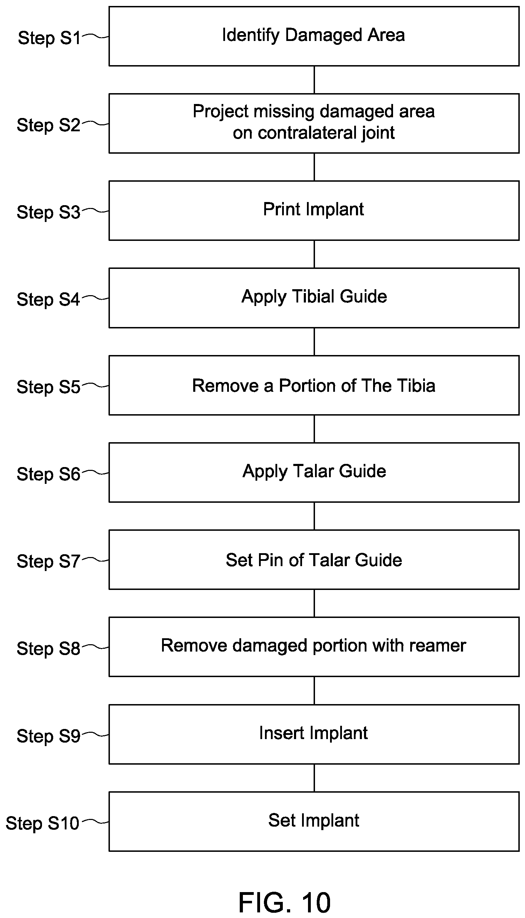

FIGS. 9A -9F show the method for preparing and implanting the talus implant 20 into a patient. FIG. 10 further shows as a flow chart the example steps for the fabrication and implantation method for the implant 20 into the talus 11. For example, the method process may start at S1 during which the health care professional may identify the most damaged area of the talus 11. Once the damaged area is identified either through surgery or through an X-ray, MRI or CT scan, the extent of the damage of this region is assessed. The health care professional may expand the area that the implant 20 will replace beyond the damaged area by a pre-set amount. The method at S2 may include the health care professional projecting the missing or damaged area based upon a mirror view or image or projection of an undamaged contralateral talus. Next, at S3, the implant 20 may be printed, followed for example by S4, where the health care professional may remove damaged bone from the tibia by using a cutting instrument. This is shown at FIGS. 9A and 9B. It should be noted that S4 and S5 may be interchanged depending on the preference of the health care professional.

The health care professional may then apply the tibial guide 70 as shown in FIG. 9A and 9B S4, wherein the tibial guide can be inserted on to the tibia 30. Pins 110, 111, 112 and 113 are inserted into the respective holes 73.1 and 73.2 as well as holes 78 and 79. With the pins being inserted into these holes the tibial guide 70 is fixed to the tibia 30. The fixing of the tibial guide 70 allows for the extension of the slot 74 (See FIG. 6D) across the tibia 30 which allows for the cutting of the tibia S5 to expose the talus to the heath care professional.

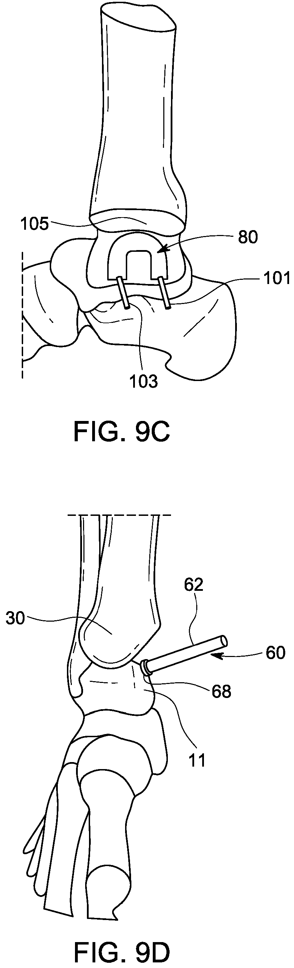

The method may include the application of the talar guide 80 by the health care professional S6 on to the talus 11 as shown in FIG. 9C. This is accomplished by placing the talar guide 80 having the surface 89 (See FIG. 7B) on the talus 11 and then inserting pins or rods such as pins 101, 103, and 105 through respective holes 82, 83, 84 (See FIG. 7C). The application of the talar guide 80 can be used to set a pin such as pin 105 which inserts through hole 82 on the talar guide 80 to fix the location of the guide 80 for the insertion of the reamer 60, 90 as listed at S7 of FIG. 10.

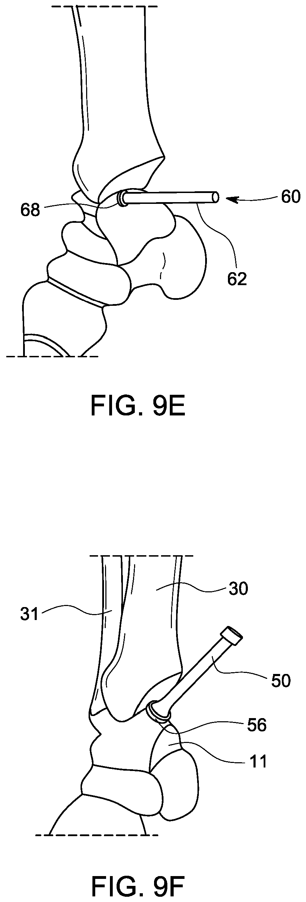

Next, as shown in FIGS. 9D and 9E and in FIG. 10, S8, the reamer 60, 90 is positioned to be inserted into the talus 11. Specifically, reamer 60 has a substantially frusto conical shaped head which closely approximates the implant 20, is inserted into the talus to remove the damaged bone and allow for the insertion of the implant 20. The reamer also includes the central hole 66, 96 which can be slid down on the pin 105 to then remove the damaged bone in a guided manner.

Once the damaged bone has been removed, at S9, the implant 20 can then be inserted with an impactor 50 to set the implant 20 into the talus. (See FIG. 9F and S9, 10 in FIG. 10). The impactor 50 has a concave face 56 that eliminates any damage to the domed surface of the implant 20. After the implant has been implanted, the procedure may be completed and the patient's incision may be closed.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the architectural and operational embodiments described herein are exemplary of a plurality of possible arrangements to provide the same general features, characteristics, and general system operation. Modifications and alterations will occur to others upon a reading and understanding of the preceding detailed description. It is intended that the invention be construed as including all such modifications and alterations that remain within the scope of the claims.

## Claims

1. A talar implant comprising:
a body section (20) that is semi-spherical in shape;
a mesh section (22); and
a plurality of solid prongs (26, 28) extending down from the bottom of the body section (20), the plurality of solid prongs (26, 28) comprising spike ends, **characterised in that** the body section comprises an exposed, smooth continuous top surface (24), wherein the mesh section extends down from a bottom side of the body section and the plurality of solid prongs extends through the mesh section.

2. The talar implant of claim 1, wherein the exposed, smooth continuous top surface (24) of the body section (20) matches a portion of the articular surface of a process portion of a talus bone.

3. The talar implant of claim 1, wherein the mesh section (22) is configured as a honeycomb pattern comprising a plurality of cells.

4. The talar implant of claim 1, wherein the mesh section (22) has a variable porosity.

5. The talar implant of claim 1, wherein the mesh section (22), the body section (20) and the plurality of solid prongs (26, 28) are printed as one piece.

6. The talar implant of claim 1, wherein the plurality of solid prongs (26, 28) are a plurality of spike elements.

7. The talar implant of claim 1, wherein the plurality of solid prongs (26, 28) comprises a plurality of spike elements extending out from the bottom side of the body section (20) and through the mesh section (22) such that the spike ends are positioned past the mesh section (22).

8. The talar implant of claim 1, wherein the mesh section (22) tapers toward the spike ends as it extends down from the body section (20).

9. The talar implant of claim 1, wherein the mesh section (22) tapers as it extends down from the body section (20).

10. The talar implant of claim 9, wherein the mesh section (22) tapers to the spike ends as it extends down from the body section (20).

11. The talar implant of claim 1, wherein the top surface (24) of the body section (20) is convex and the bottom side of the body section (20) is concave.

## Patentansprüche

1. Ein Talusimplantat, das Folgendes beinhaltet:
einen Körperabschnitt (20), der halbkugelförmig ist;
einen Netzabschnitt (22); und
eine Vielzahl von massiven Zinken (26, 28), die sich von dem unteren Bereich des Körperabschnitts (20) nach unten erstrecken, wobei die Vielzahl von massiven Zinken (26, 28) Spitzenenden beinhalten, **dadurch gekennzeichnet, dass** der Körperabschnitt eine freiliegende, glatte, durchgehende Oberfläche (24) beinhaltet, wobei sich der Netzabschnitt von einer unteren Seite des Körperabschnitts nach unten erstreckt und sich die Vielzahl von massiven Zinken durch den Netzabschnitt erstrecken.

2. Talusimplantat gemäß Anspruch 1, wobei die freiliegende, glatte, durchgehende Oberfläche (24) des Körperabschnitts (20) mit einem Teil der Gelenkfläche eines Fortsatzteils eines Talusknochens übereinstimmt.

3. Talusimplantat gemäß Anspruch 1, wobei der Netzabschnitt (22) als ein Wabenmuster konfiguriert ist, das eine Vielzahl von Zellen beinhaltet.

4. Talusimplantat gemäß Anspruch 1, wobei der Netzabschnitt (22) eine variable Porosität aufweist.

5. Talusimplantat gemäß Anspruch 1, wobei der Netzabschnitt (22), der Körperabschnitt (20) und die Vielzahl von massiven Zinken (26, 28) als ein Stück gedruckt sind.

6. Talusimplantat gemäß Anspruch 1, wobei die Vielzahl von massiven Zinken (26, 28) eine Vielzahl von Spitzenelementen sind.

7. Talusimplantat gemäß Anspruch 1, wobei die Vielzahl von massiven Zinken (26, 28) eine Vielzahl von Spitzenelementen beinhalten, die sich von der unteren Seite des Körperabschnitts (20) nach außen und durch den Netzabschnitt (22) erstrecken, sodass die Spitzenenden über den Netzabschnitt (22) hinaus positioniert sind.

8. Talusimplantat gemäß Anspruch 1, wobei sich der Netzabschnitt (22) in Richtung der Spitzenenden verjüngt, während er sich von dem Körperabschnitt (20) nach unten erstreckt.

9. Talusimplantat gemäß Anspruch 1, wobei sich der Netzabschnitt (22) verjüngt, während er sich von dem Körperabschnitt (20) nach unten erstreckt.

10. Talusimplantat gemäß Anspruch 9, wobei sich der Netzabschnitt (22) bis zu den Spitzenenden verjüngt, während er sich von dem Körperabschnitt (20) nach unten erstreckt.

11. Talusimplantat gemäß Anspruch 1, wobei die Oberfläche (24) des Körperabschnitts (20) konvex ist und die untere Seite des Körperabschnitts (20) konkav ist.

## Revendications

1. Un implant talaire comprenant :
une section de corps (20) qui est de forme semi-sphérique ;
une section à mailles (22) ; et
une pluralité de picots pleins (26, 28) s'étendant vers le bas à partir du dessous de la section de corps (20), la pluralité de picots pleins (26, 28) comprenant des extrémités en pointe, **caractérisé en ce que** la section de corps comprend une surface de dessus continue lisse exposée (24), dans lequel la section à mailles s'étend vers le bas à partir d'une face de dessous de la section de corps et la pluralité de picots pleins s'étend à travers la section à mailles.

2. L'implant talaire de la revendication 1, dans lequel la surface de dessus continue lisse exposée (24) de la section de corps (20) correspond à une portion de la surface articulaire d'une portion de processus d'un talus.

3. L'implant talaire de la revendication 1, dans lequel la section à mailles (22) est configurée comme un motif en nid d'abeilles comprenant une pluralité de cellules.

4. L'implant talaire de la revendication 1, dans lequel la section à mailles (22) a une porosité variable.

5. L'implant talaire de la revendication 1, dans lequel la section à mailles (22), la section de corps (20) et la pluralité de picots pleins (26, 28) sont imprimées en une seule pièce.

6. L'implant talaire de la revendication 1, dans lequel la pluralité de picots pleins (26, 28) est une pluralité d'éléments en pointe.

7. L'implant talaire de la revendication 1, dans lequel la pluralité de picots pleins (26, 28) comprend une pluralité d'éléments en pointe s'étendant vers l'extérieur à partir de la face de dessous de la section de corps (20) et à travers la section à mailles (22) de telle sorte que les extrémités en pointe sont positionnées au-delà de la section à mailles (22).

8. L'implant talaire de la revendication 1, dans lequel la section à mailles (22) s'effile en direction des extrémités en pointe alors qu'elle s'étend vers le bas à partir de la section de corps (20).

9. L'implant talaire de la revendication 1, dans lequel la section à mailles (22) s'effile alors qu'elle s'étend vers le bas à partir de la section de corps (20).

10. L'implant talaire de la revendication 9, dans lequel la section à mailles (22) s'effile jusqu'aux extrémités en pointe alors qu'elle s'étend vers le bas à partir de la section de corps (20).

11. L'implant talaire de la revendication 1, dans lequel la surface de dessus (24) de la section de corps (20) est convexe et la face de dessous de la section de corps (20) est concave.
